# EUROPEAN PATENT APPLICATION

(11) **EP 2 352 116 A1**
(43) Date of publication of application: **03.08.2011**
(21) Application number: 08877545.7
(22) Date of filing: 22.10.2008
(51) Int. Cl.: G06Q 50/00

(54) **INFORMATION MANAGEMENT SUPPORT METHOD, MANAGEMENT INFORMATION VISUALIZATION DEVICE, INFORMATION MANAGEMENT SYSTEM, AND MANAGEMENT INFORMATION VISUALIZATION METHOD**

(71) Applicant: Hitachi, Ltd., Chiyoda-ku Tokyo 100-8280 (JP)
(72) Inventor: ISHII, Tomoyuki, Tokyo 100-8280 (JP); SATO, Keiichi, Tokyo 140-0002 (JP)
(74) Representative: MERH-IP Matias Erny Reichl Hoffmann
(86) International application number: PCT/JP2008/069158
(87) International publication number: WO 2010/046979

(57) **Abstract**

A management information visualization device (management provider server 30) according to the present invention includes: an information registration management unit (information management unit 304) that registers service information transmitted from a service providing device (service provider server 20), associating the service information with user identification information and service content identification information which are related to the service information; and an information input/output unit (user GUI providing unit 303) that receives, from a user device, a request for viewing service information, creates management information (a top screen) including a service content list associated with service content identification information and transmits the management information to the user device, and, when one of the service content list displayed on the user device being selected by operation, searches in a management information DB300 and transmits corresponding service information to the user device.

## Description

### Technical Field

The present invention relates to a technology for management and visualization of health management information and the like.

### Background Art

Health management information including height, weight, blood pressure, and blood glucose level and biological information including genetic information on the array and type and the like of genes are information of an extremely high value to recognize the physical state of a person. However, when such information is coupled with personal information, such as name, address, telephone number, electronic mail address, which enables identification of a person, then the information is handled as sensitive information.
Incidentally, the law related to the protection of personal information (the Personal Information Protection Law in Japan) stipulates 'According to this law, "personal information" refers to information that is related to a living person and enables identification of a specific person by name, birth date, and other description included in the information (including information that can be easily checked with other information to enable identification of a specific person).' (Article 2 of the law).

On the other hand, according to JIS Q15001, sensitive information refers to several items including (1) matters related to ideology, belief, and religion, (2) matters related to race, ethnic, ancestry, permanent domicile (excluding information on prefectural governments of location), physical or psychiatric disability, criminal record, and matters which cause other social discrimination, (3) matters related to solidarity right of workers, collective bargaining, and actions on other group activities, (4) matters related to participation in group demonstration, exercise of right of petition, and exercise of other political rights, (5) insurance, medical, and sexual behavior. Further, information other than these that can be sensitive information exists, and genetic Information, appearance, hobby and favorite can be sensitive information. However, there is no clear definition in reality.

However, leakage of sensitive information possibly causes a significant loss to the social life, based on the information, of a person. Further, if personal information has been obtained together with information, such as gene genetic Information, which does not change in all the life of the person, information with an extreme risk for the person may be provided to a business company. Accordingly, particular care should be taken in handling such information.

From this point of view, 'the guideline for protection of personal information in business fields using personal genetic information, among economical and industrial fields', with exception of the case based on the laws or ordinances of personal genetic information applied to business, prohibits obtaining or using sensitive information, and most local governments having personal information protection codes have established stipulations related to sensitive information. Further, 'the directive, of European Parliament and European Council (EU Directive), regarding protection of individuals related to personal data processing and free transfer of it' stipulates 'Members prohibit processing of personal data that leaks information on race, ethnic, political view, religion, ideology, sentiment, and participation in a labor union, and processing of data related to health or sexual behavior'. In such a manner, as various constraints exist on handling sensitive information, not only it requires a significant effort to meet them, but also it is sometimes difficult to provide a service that uses sensitive information.

In this situation, patent applications have been made for logistic and sales service systems that enable a user to request a supplier for a commodity or a service while keeping personal information on the user secret (for example, refer to Patent Document 1).
According to the technology disclosed by Patent Document 1, an anonymous delivery support server issues an anonymous ID corresponding to a user ID (identification), and stores a name and an address corresponding to the user ID, associating them with the anonymous ID. Then, when the user requests a supplier to deliver a commodity, the user inputs the anonymous ID to a supplier instead of the delivery destination, using a terminal, and the supplier transmits the anonymous ID having been input to the supplier support server, to a home delivery service provider. Further, a terminal of the delivery service provider obtains the name and the address corresponding to the received anonymous ID from an anonymous delivery support server and delivers the commodity of the supplier to the name and the address. Thus, information of individuals is protected.
Patent Document 1: JP 2003-296637 A (paragraph 0007, FIG. 1)

### Disclosure of the Invention

### Problem to be Solved by the Invention

In order to provide a secure and safe service using sensitive information, described above, it is important to manage data, separating 'personal information' and 'information (semi-sensitive information) that turns into sensitive information when coupled with personal information' so that these two kinds of information are prevented from coupling together. In real operation, it is desirable that a database that accumulates personal information and a database that accumulates semi-sensitive information are separated from each other. Based on this, it is further important to control viewable information viewer by viewer to prevent coupling between personal information and semi-sensitive information, and thereby provide a requested service without handling sensitive information that enables identification of a person.
However, according to the technology disclosed by Patent Document 1, described above, as an anonymous delivery support server has both anonymous IDs and personal information, it is not possible to provide a requested service without identifying a person.

The present invention has been developed to solve the above-described problem, and an object of the invention is to provide a technology for providing a requested service by handling semi-sensitive information and personal information being separated from each other, and to provide a user-friendly interface for a viewer.

### Means for Solving the Problem

To solve the above-described problem, according to the present invention, a management information visualization device is connected via a network with user devices and a service providing device that receives a request/requests for service provision from a user device/devices and obtains personal information including user identification information. The management information visualization device registers service information provided by the service providing device, into a database together with service content identification information assigned uniquely to each individual service that a user has contracted. The management information visualization device includes an information registration management unit and an information input/output unit.
In the above-described configuration, the information registration management unit registers service information transmitted from the service providing device into the database, associating service information with user identification information and service content identification information which are related to this service information. The information input/output unit receives, from a user device, a request for viewing service information, the request being based on either user identification information or service content identification information or based on a combination of these; creates management information including a service content list associated with service content identification information and transmits the management information to the user device having made the request for viewing service information; and, when one from the service content list displayed on the user device being selected by operation via the user device, searches in the database and transmits corresponding service information to the user device having made the request for viewing service information. Advantages

According to the present invention, it is possible to provide a technology for providing a requested service, such as to handle semi-sensitive information and personal information to be separated from each other, and providing a user-friendly interface for a viewer.

### Brief Description of the Drawings

FIG. 1 is a diagram for illustrating business entities and the relationships between the entities, according to a method for visualization of management information in an information management system in the present embodiment;
FIG. 2 is a sequence diagram showing a flow of information between the business entities, according to the method for visualization of management information in the information management system in the present embodiment;
FIG. 3 is a diagram showing an example of a system configuration of the information management system in the present embodiment;
FIG. 4 is a sequence diagram showing the operation of the information management system in the present embodiment;
FIG. 5 is a diagram that includes a block diagram showing the inner configuration of a service provider server and a block diagram showing that of a management provider server used in the information management system in the present embodiment;
FIG. 6 is a diagram that includes a block diagram showing the inner configuration of a management provider server for user terminals and testing devices used in the information management system in the present embodiment;
FIG. 7 is a diagram showing an example of a data structure of a personal information DB used in the information management system in the present embodiment;
FIGS. 8A and 8B are diagrams showing an example of a data structure of the management information DB of the management information visualization device in the present embodiment;
FIGS. 9A and 9B are diagrams showing an example of user IDs, service IDs, and service information which are registered in the management information DB of the management information visualization device in the present embodiment;
FIG. 10 is a diagram showing an example of a data structure of semi-sensitive information registered in the management DB of the management information visualization device in the present embodiment;
FIGS. 11A and 11B are diagrams showing an example of the data structure of a view control information table of the management information visualization device in the present embodiment;
FIG. 12 is a flowchart showing the operation of the management information visualization device in the present embodiment;
FIG. 13 is a diagram showing an example of the screen configuration of a top screen displayed on a user terminal by the management information visualization device in the present embodiment;
FIG. 14 is a diagram showing an example of the screen configuration of a practice card list screen displayed on the user terminal by the management information visualization device in the present embodiment; and
FIG. 15 is a diagram showing an example of the screen configuration of a personal setting screen displayed on the user terminal by the management information visualization device in the present embodiment.

### Reference Numerals

1...end user, 2...health service provider, 3... data collection and keeping service provider, 4...testing device maker, 10...user terminal (user device), 20...service provider server (service providing device), 30...management provider server ( management information visualization device ), 40...testing device, 50...network, 200...personal information DB, 201...service ID issuing unit, 202...personal information management unit, 203...data display unit, 204...data output unit (transmission means), 205...data search unit, 206...testing data input unit, 300...management information DB (database), 301...user management unit, 302...service management unit, 303...user GUI providing unit (information input/output means), 304...information management unit (information registration/management means), 305...ID conversion information management unit, 306...provider data input/output unit (data input/output unit communicating with service provider side)

### Best Mode for Carrying Out the Invention

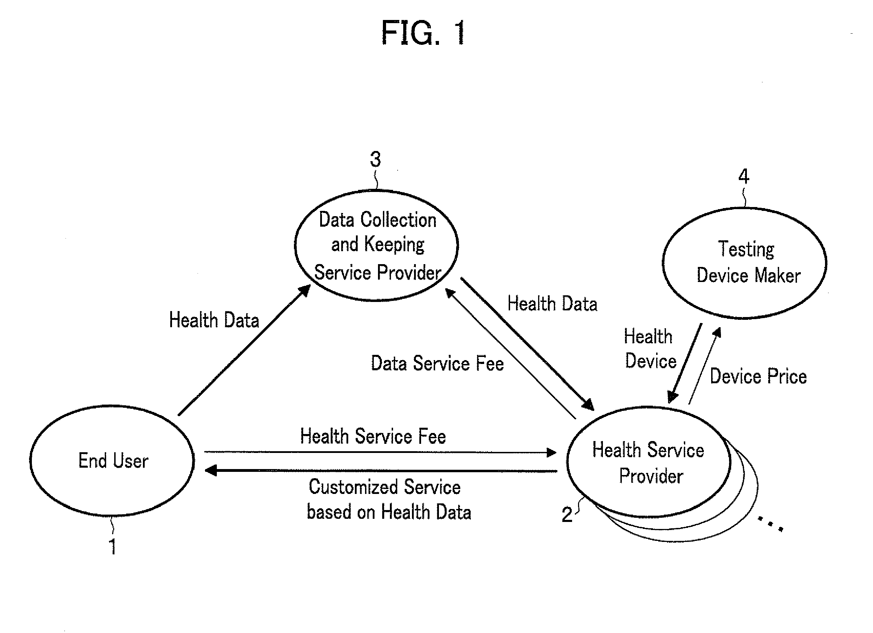

A best mode (hereinafter, referred to as 'embodiment') for carrying out the present invention will be described below, referring to the drawings.
Description of Method for Visualization of Management Information in Information Management System
FIG. 1 is a diagram for illustrating business entities and the relationships between the entities, according to a method for visualization of management information in an information management system in the present embodiment.

In FIG. 1, a data collection and keeping service provider 3 collects and keeps health data (health management information) of an end user 1 via a network. However, the data collection and keeping service provider 3 only keeps health data but does not keep personal information, such as the name and the address. A plurality of health service providers 2 rent a testing device to the end user 1, take a view of at least a part of the health data that the data collection and keeping service provider 3 keeps, provide a customized service, based on the health data, to the end user 1, and receive a fee (health service fee). Herein, services that the health service providers 2 provide are assumed to be a dietary instruction service for dietary instruction, based on the constitution including weight transition and genetic information, and exercise instruction service, such as a service at a fitness club.

Testing device makers 4 sell testing devices, such as a health sensor, a weight scale, a body fat scale, a pedometer to the health service providers 2, and receive payment for the devices. Incidentally, the end user 1 may purchase a testing device not through the health service provider 2.

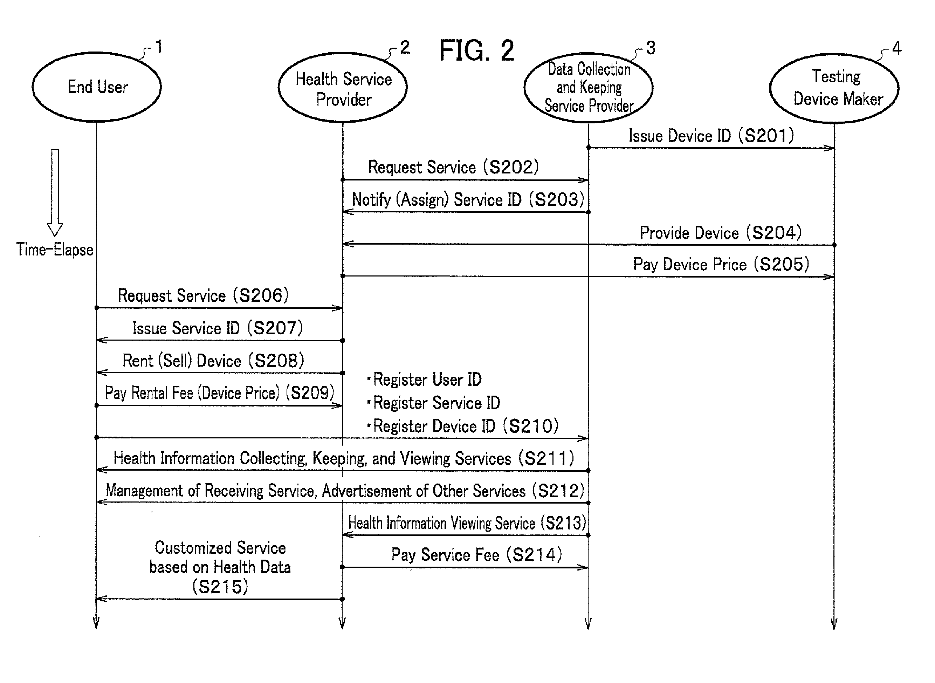

FIG. 2 is a sequence diagram showing a flow of information between the business entities, according to the method for visualization of management information in the information management system in the present embodiment.
The flow of information between the business entities, shown in FIG. 1, will be briefly described below, referring to the sequence diagram shown in FIG. 2.

First, the data collection and keeping service provider 3 issues device IDs unique to each individual testing device provided by a testing device maker 4 (step S201).
A health service provider 2 requests the data collection and keeping service provider 3 for a service (step S202). Upon receiving this request, the data collection and keeping service provider 3 assigns service IDs (service content identification information) for the health service provider 2 having made the request, and issues the service IDs in a set (a set of plural IDs) (step S203). Individual service IDs are unique, which enables management of the kinds of services, health service providers 2, the ranges of viewable data, effective validities, fees, and the like. Further, herein, it is assumed that the data collection and keeping service provider 3 issues service IDs in a set in advance, however, the data collection and keeping service provider 3 may issue an service ID after a request for a service has been made by the end user 1.

On the other hand, a testing device maker 4 provides the health service provider 2 with testing devices (step S204), and the health service provider 2 pays for the devices to the testing device maker 4 (step S205). Incidentally, manners of providing a testing device to the end user 1 include rental and selling by the health service provider 2 and selling by the testing device maker 4.

Then, the end user 1 requests the health service provider 2 for a service (step S206), and upon receiving this request, the health service provider 2 issues one of the service IDs assigned in advance by the data collection and keeping service provider 3, to the end user 1 who has made the request (step S207). The health service provider 2 also rents or sells, to the end user 1, a testing device procured from the testing device maker 4, (step S208). In response, the end user 1 pays the service fee including the rental fee or the price of the testing device (step S209).

Subsequently, the end user 1 registers a user ID, a service ID, and a device ID to the data collection and keeping service provider 3 (step S210). In such a manner, the end user 1 can enjoy the service, provided by the data collection and keeping service provider 3, of collection, keeping, and viewing of the health data of himself/herself (step S211). Further, the end user 1 also can obtain (manage) a service currently received from the health service provider 2, and view advertisements of other services and the like (step S212).
Incidentally, once the end user 1 has registered a user ID, a service ID, and a device ID, the data collection and keeping service provider 3 thereafter performs management, associating the user ID, the service ID and the device ID. Consequently, the end user 1 can issue a request for viewing service information, based on these IDs.

On the other hand, the health service provider 2 issues a request to the data collection and keeping service provider 3 for viewing health data and refers to at least a part of the health data of the end user 1 (step S213), and pays a service fee to the data collection and keeping service provider 3 (step S214). Further, the health service provider 2 provides the end user 1 with service information having been customized, based on the health data (step S215).

### Description of Information Management System

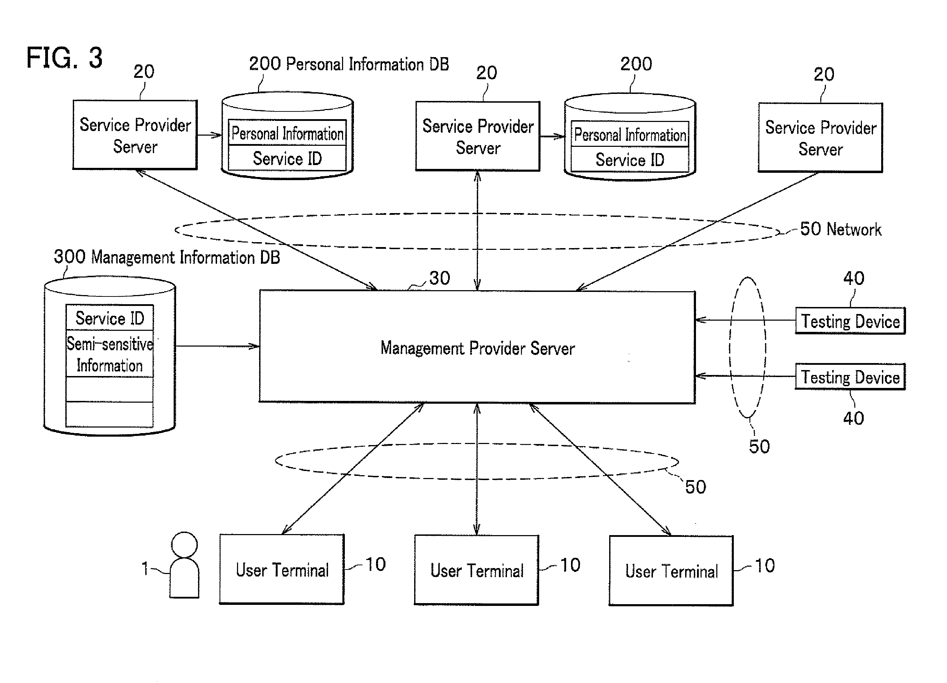

FIG. 3 is a diagram showing an example of the system configuration of the information management system in the present embodiment.
As shown in FIG. 3, the information management system in the present embodiment is configured by (directly or indirectly) connecting a user terminal 10 as a user device configured for example with a personal computer, a service provider server 20 as a service providing device, a management provider server 30 as a management information visualization device, and a testing device 40 via a network 50, such as an IP (Internet Protocol) net. Incidentally, the service provider server 20 and the management provider server 30 can be implemented by computer device provided with a CPU (Central Processing Unit), a RAM (Random Access Memory), a ROM (read Only Memory), a hard disk, various interfaces, and the like.

User terminals 10 are a user devices used by the end user 1 who receives services, such as physical information measuring. Further, service provider servers 20 are service providing devices that receive requests (requests for a service) from the user terminals 10 for testing of a specimen or the like, are owned by plural individual health service providers 2 who execute testing, and are provided with respective personal information DBs (Data Bases) 200. Incidentally, there may be a case that some service provider servers 20 are not provided with a personal information DB 200 (for example, the service provider server 20 at the right end portion of FIG. 3).
The management provider server 30 is a management information visualization device provided with a management information DB (Data Base) 300 that stores the data of a testing result, separating the data from the personal information, such as the name, of a user (the end user 1, also merely referred to as 'user').

A testing device 40 may be, for example, a blood-pressure gauge, a weight scale, a mobile sensor of a wrist watch type with an incorporated ratio communication medium, or the like. For example, if a testing device 40 is a mobile sensor, it is assumed that a user always attaches the mobile sensor to a wrist watch, counts the number of swinging an arm along a temporal axis by an incorporated three-axis acceleration sensor, and supplies (transmits) measurement data as a result to the management provider server 30 wirelessly via a network 50. Further, a testing device 40 may be arranged with connection such that the testing device 40 communicates with the user terminal 10 via the incorporated radio communication medium and supplies measured data via the user terminal 10 to the management provider server 30.

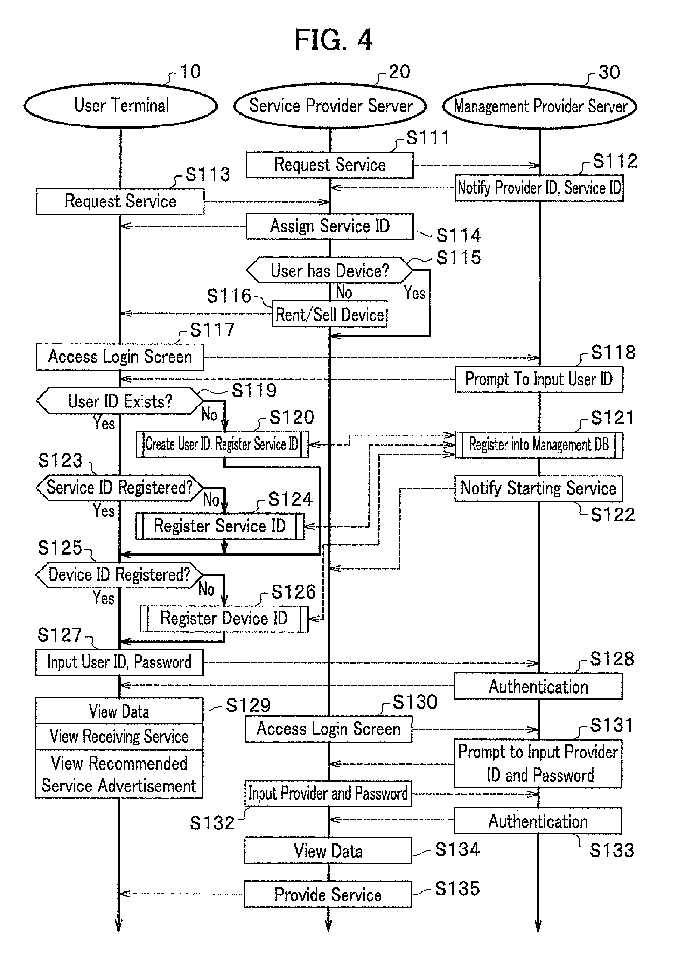

FIG. 4 is a sequence diagram showing the operation of the information management system in the present embodiment, and shows the flow of data between the user terminal 10, the service provider server 20, and the management provider server 30, and shows the outlines of the respective processes in these.
The operation of the information management system in the present embodiment will be described below, referring to the sequence diagram in FIG. 4.

First, the service provider server 20 issues a service request (herein an application for a service) to the management provider server 30 (step S111), and upon receiving this request, the management provider server 30 issues a provider ID assigned to an individual service provider and plural service IDs assigned in a set to an individual service, and notifies these to the service provider server 20 which has made the request (step S112).

The user terminal 10 issues a service request (an application for a service) to the service provider server 20 (step S113), and upon receiving this request, the service provider server 20 assigns one of the services IDs already having been issued by the management provider server 30 to the user terminal 10 (step S114).

Subsequently, the service provider server 20 inquires the user terminal 10 whether or not the user has a testing device 40. If it is recognized by a reply that the user does not have a testing device 40 (Step S115 'No'), then the service provider server 20 instructs the health service provider to rent or sell a testing device 40 (step S116).

On the other hand, when the user terminal 10 has accessed the login screen on the management provider server 30 (step S117), the management provider server 30 prompts the user terminal 10 for input of a user ID (step S118). The user terminal 10 determines whether or not a user ID has been registered (step S119). If not yet registered (step S119 'No'), then the user terminal 10 creates a user ID and registers the service ID (step S120). Incidentally, the user has been notified of the service ID in the above-described step S114.

If a user ID has already been registered (step S119 'Yes'), the user terminal 10 determines whether or not the service ID has been registered (step S123). If not yet registered (step S123 'No'), then the user terminal 10 registers the service ID having been already notified (step S124).

Subsequently, the user terminal 10 determines whether or not the device ID of the testing device 40 has been registered (step S125). If not yet registered (step S125 'No'), the user terminal 10 registers the device ID (step S126).
The user ID, the service ID, and the device ID registered here are then registered by the management provider server 30 into the management information DB 300 with association therebetween (step S121), and thereafter the service of collection and keeping of and providing information of the health data of the user is available, and this is notified to the service provider server 20 (step S122).

Following the above-described preparation work, the user terminal 10 issues, to the management provider server 30, a request for viewing service information, based on the user ID and a password input, or the service ID associated with the user ID (step S127). Upon receiving this request, the management provider server 30 performs user authentication (step S128), and when authentication has been established, the user becomes able to view health data, a service received from the health service provider, advertisements of recommended services, and the like (step S129). Incidentally, the password is assumed to be created together with the user ID when the user ID is created in step S120, and then notified to the user.

On the other hand, when the service provider server 20 has accessed via a login screen to the management provider server 30 (step S130), the management provider server 30 prompts input of a provider ID and a password (step S131). If the service provider inputs a provider ID having been informed in advance and a password to the service provider server 20 (step S132), then the management provider server 30 performs authentication of the service provider (step S133).
Herein, if the authentication is established, then the service provider server 20 is permitted to view the health data of the user (step S134), and provides a service that is customized, based on the viewed heath data, to the user terminal 10 (step S135).

### Description of Service Providing Device (Service Provider Server)

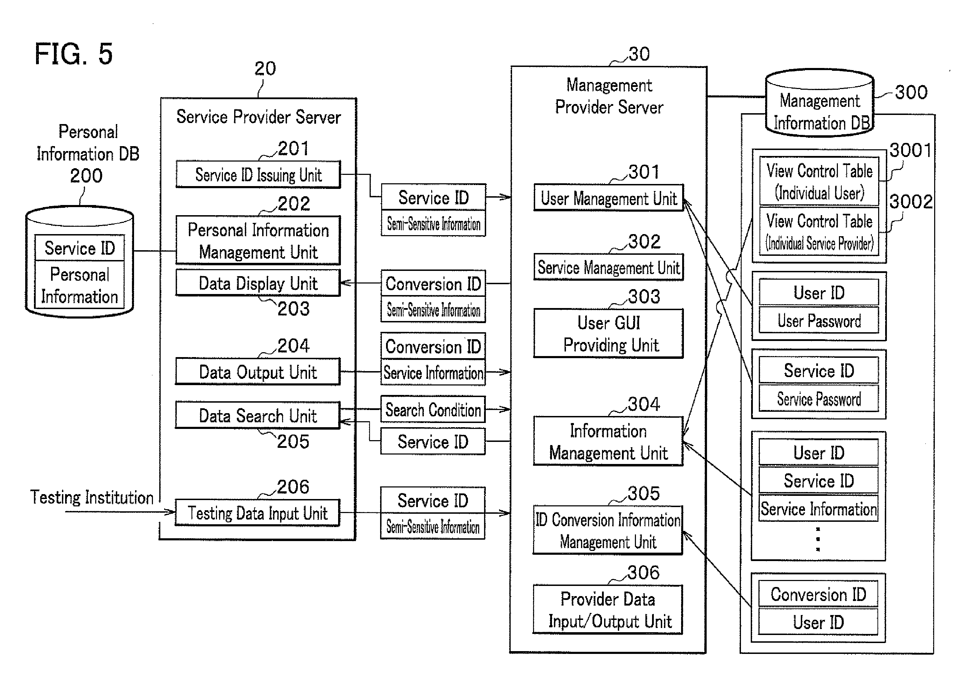

FIG. 5 is a diagram that includes a block diagram showing the inner configuration of the service provider servers 20 and a block diagram showing that of the management provider server 30 shown in FIG. 3.
As shown in FIG. 5, the service provider server 20 includes a service ID issuing unit 201, a personal information management unit 202, a data display unit 203, a data output unit 204, a data search unit 205, and a testing data input unit 206.

The service ID issuing unit 201 has a function to issue a service ID for an individual service content upon application for a service via a user terminal 10 (step S113 in FIG. 4).
The personal information management unit 202 has a function to manage personal information that is obtained upon application for a service by a user via the user terminal 10 and then stored into the personal information DB 200.
The data search unit 205 has a function to search data, which satisfies a search condition, from a management information DB 300, and the data display unit 203 has a function to display semi-sensitive information and the like obtained by the search.

The data output unit 204 has a function to transmit service information, such as an advice regarding semi-sensitive information and the like, to the management provider server 30. When service information is registered into the management information DB 300 of the management provider server 30, the data output unit 204 function as 'transmitting means' that adds a user ID and a service ID, which are related to the service information, to the service information, and transmits the service information via the network 50 to the management provider server 30.
The testing data input unit 206 has a function to add a service ID to testing data ((semi-) sensitive information) obtained from an external testing institution, and transmit the testing data to the management provider server 30.

### Description of Management Information Visualization Device

### (Management Provider Server)

As shown in FIG. 5, the management provider server 30 includes a user management unit 301, a service management unit 302, a user GUI (Graphical User Interface) providing unit 303, an information management unit 304, an ID conversion information management unit 305, and a provider data input/output unit (a data input/output unit communicating with service provider side) 306.
The user management unit 301 has a function to manage user IDs and passwords which are registered for individual users in the management information DB 300. The service management unit 302 has a function to manage service IDs and service passwords registered in the management information DB 300. The user management unit 301 and the service management unit 302 also have a function to manage respective view control information tables 3001 and 3002 in which data viewable respectively to individual users and individual service providers are set. The data structures and the details of the view control information tables 3001 and 3002 will be described later.

The user GUI providing unit 303 has a function to perform transmission/receiving of data to/from user terminals 10. The user GUI providing unit 303 has a function as 'information input/output means' for receiving a request for viewing service information, based on either a user ID or a service ID or based on combination of the both, via the network 50 from a user terminal 10; creating management information including a service content list (a later-described card thumbnail list) associated with service IDs and transmitting the management information to the user terminal 10 having made the request for viewing service information; and upon selection with operation, via the user terminal 10, of one from the service content list displayed on the user terminal 10, searching the selected service information in the management information DB 300 and transmitting the service information via the network 50 to the user terminal 10 having made the request for viewing service information. Details will be described later.

The information management unit 304 has a function to manage data related to service contents which are registered in the management information DB 300. The information management unit 304 functions as 'information registration management means' for associating service information with a user ID and a service ID which are related to the service information and registering service information transmitted from the service provider server 20.
Incidentally, the ID conversion information management unit 305 has a function, in providing semi-sensitive information to a service provider server 20 or a user terminal 10, to disguise a user ID attached to the semi-sensitive information as another user ID in order to hide as to whom the semi-sensitive information belongs to. The provider data input/output unit 306 has a function to transmit/receive data to/from service provider servers 20.

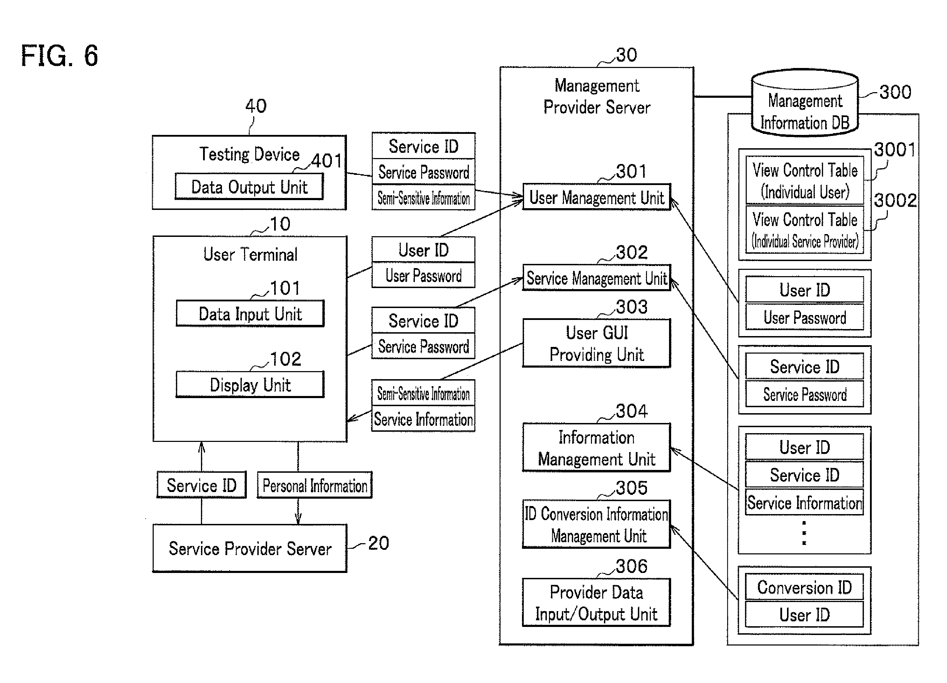

FIG. 6 is a diagram that includes a block diagram showing the inner configurations of user terminals 10, testing devices 40, and the management provider server 30, shown in FIG. 3.
As shown in FIG. 6, a user terminal 10 includes a data input unit 101 for input of a user ID, a user password, and the like to the management provider server 30, and a display unit 102 for displaying data of a search result and the like received from the management provider server 30. The display unit 102 receives management information including a service content list transmitted from the management provider server 30 via the network 50, and displays the management information on a screen to prompt a user for selection with operation. Further, the user terminal 10 obtains, by the data input unit 101, information on the service content selected by the user operation, transmits the information via the network 50 to the management provider server 30, and receives corresponding service information transmitted via the network 50 from the management provider server 30 to display the service information on a screen on the display unit 102. Incidentally, a testing device 40 includes a data output unit 401 for transmitting semi-sensitive information on height, weight, and the like as a result of measurement, to the management provider server 30.

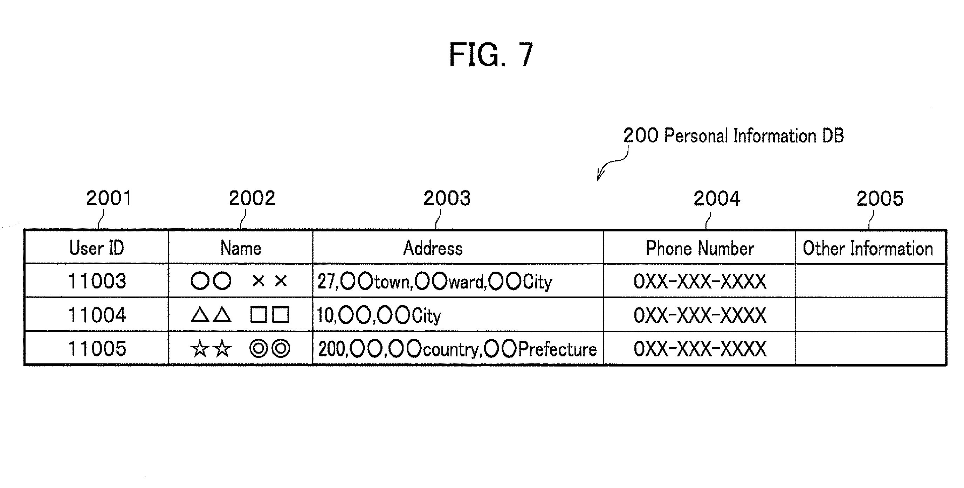

FIG. 7 is a diagram showing an example of the data structure of personal information of users registered in the personal information DB 200 of a service provider server 20, wherein each user is given with a unique user ID 2001, and information that enables identifying an individual person and includes a name 2002, an address 2003, a phone number 2004, other information 2005, and the like is registered for each individual user ID 2001.
This personal information has been input by a user himself/herself or an attendant from a window terminal when the user requested a window institution of a service provider server 20 to provide a service.

When input of the personal information including the address and the name of a user has been completed, the user management unit 301 of the management provider server 30 automatically generates a user ID 2001, and displays it on the screen on the window terminal to notify the user of the user ID 200199. The user management unit 301 registers the personal information in the personal information DB 200, associating the personal information with the user ID.

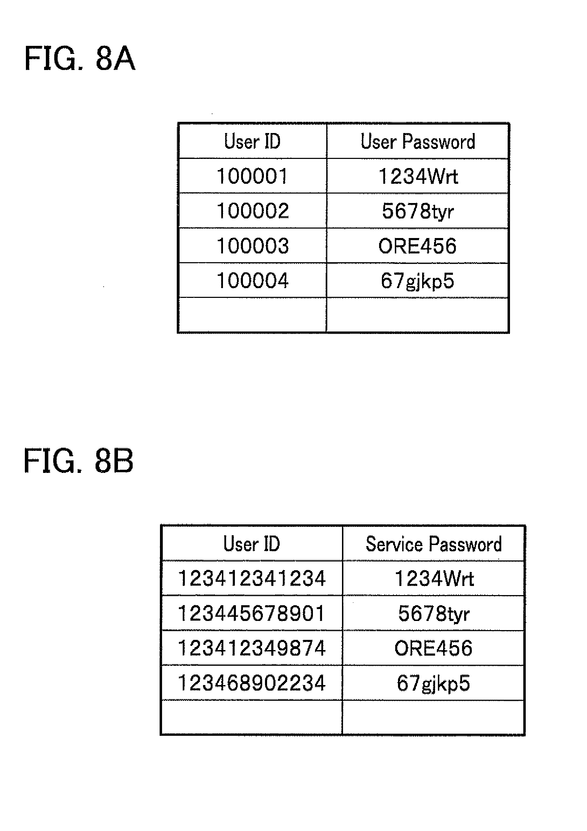

FIGS. 8A and 8B show an example of information registered in the management information DB 300 of the management provider server 30. FIG. 8A shows an example of user IDs and user passwords. FIG. 8B shows an example of service IDs and service passwords.
After obtaining a service ID from a service provider server 20, a user transmits a user ID and a user password defined by the user from a user terminal 10 to the service provider server 20, and the user management unit 301 thereby registers the user ID and the user password, as shown in FIG. 8A. This user ID and user password are used when the management provider server 30 authenticates the user of the user terminal 10. Further, a service ID and a service password, as shown in FIG. 8B, are used in registering a result of testing height, weight, and the like, using a testing device 40.

The service provider server 20 creates a service ID and a service password for each individual testing device 40. A created service ID and service password are transmitted to the service management unit 302 of the management provider server 30, and registered into the management information DB 300.
A user registers a service by associating the user ID with the service ID. The user performs testing or measuring, using a testing device 40 and transmits a result together with the service ID to the management provider server 30. The management provider server 30 searches the service ID in the service management unit 302 to recognize whether or not the service ID is registered, and if registered, the management provider server 30 registers the measurement result into the management information DB 300. A result of the registration is viewable on a screen of the user terminal 10. Thus, the user can continuously accumulate measurement results obtained by the testing device 40 in the management information DB 300. Incidentally, a service ID may be used together with the device ID of a testing device 40, or the device ID may be used instead of the service ID.

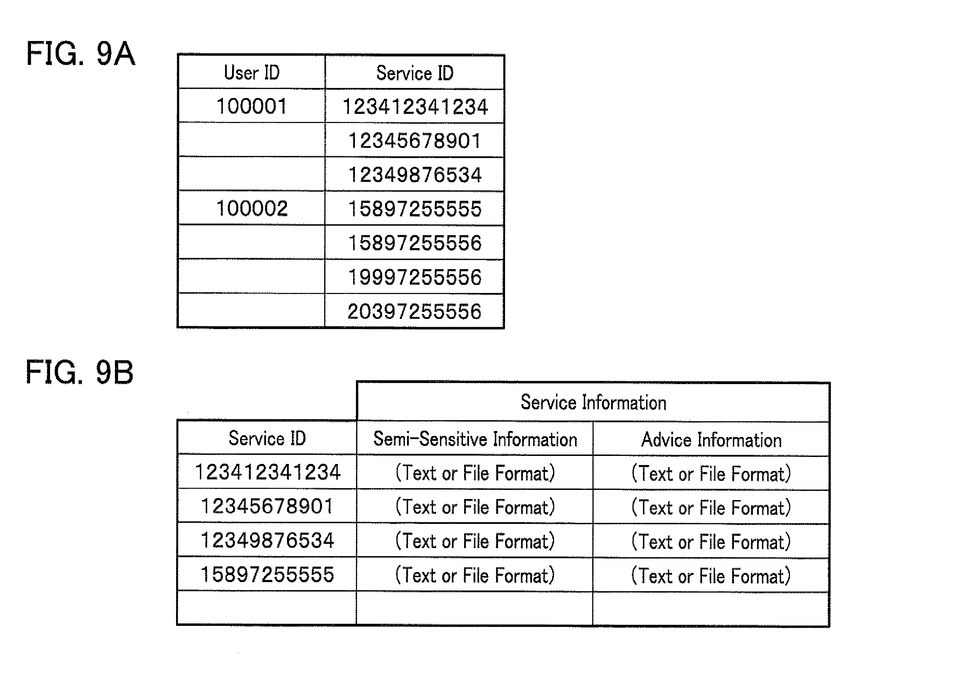

FIGS. 9A and 9B show an example of information registered in the management information DB 300. FIG. 9A shows an example of the data structure of user IDs and service IDs. FIG. 9B shows an example of the data structure of service IDs and service information.
Herein, a unique service ID is assigned to each individual service to be provided to a user, and the service ID comes to be registered in the management information DB 300, being associated with the user ID of the user to whom the service is provided. Service information representing the content of a service is registered in the management information DB 300, being associated with a service ID. The example in FIG. 9A shows that three services are provided to the user with a user ID=100001, and the contents thereof can be recognized by a view of the service information (refer to FIG. 9B) associated with respective service IDs. In FIG. 9B, the concrete contents of the service information are omitted. The table in FIG. 9A corresponds to 'information on association between users and service contents'.

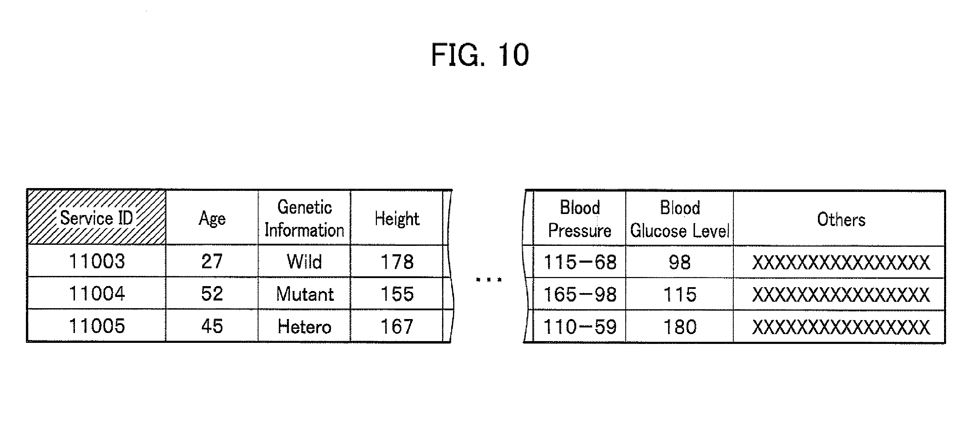

FIG. 10 is a diagram showing an example of the data structure of semi-sensitive information registered in the management DB 300.
Herein, FIG. 10 shows that semi-sensitive information, such as age, genetic information, and height, is registered for each individual service ID. This semi-sensitive information is the data of a service content of a service that provides a user with a result of testing the specimen of the user.

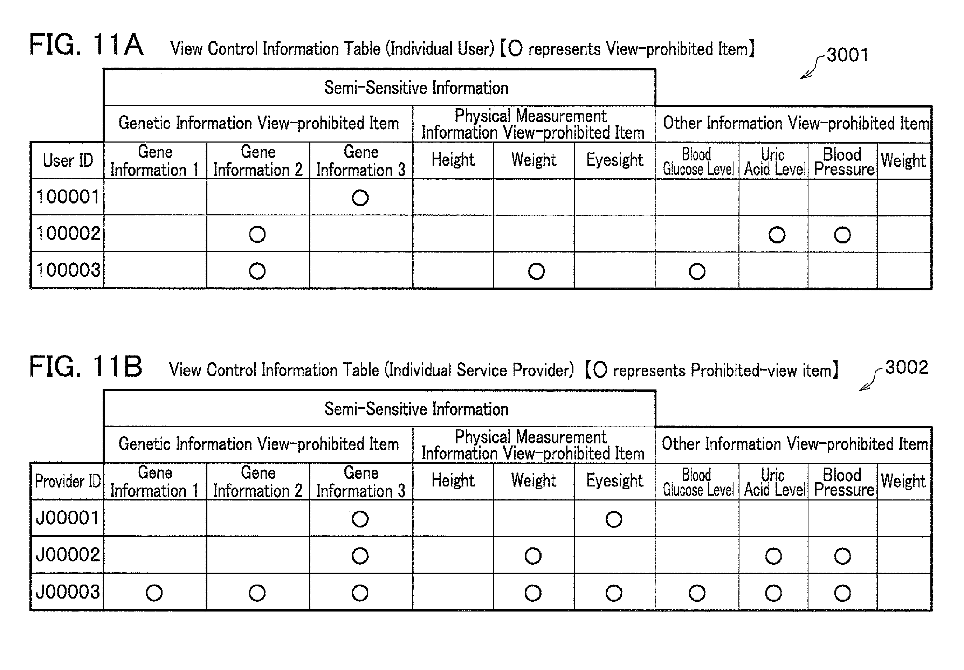

FIGS. 11A and 11B are diagrams showing an example of the data structures of view control information tables 3001 and 3002 for control of viewable data for each individual user and service provider.
In the view control information table (for individual users) 3001, shown in FIG. 11A, items which prohibit viewing (view-prohibited items) are set for individual user IDs with regard to genetic information, physical measurement information, and other information. For example, for the user with user ID=100001, prohibition of viewing 'genetic information 3' is set. Each individual user can designate which items to be prohibited from viewing, via the user terminal 10 from the point of view of self-defense.

In the view control information table (for individual service providers) 3002, shown in FIG. 11B, items which prohibit viewing (view-prohibited items) are set for individual provider IDs with regard to genetic information, physical measurement information, and other information.
For example, for the service provider with provider ID=J00001, prohibition of viewing 'genetic information 3' and 'eye sight' is set. The manager of the management provider server 30 can set which items to be prohibited from viewing. For example, the management provider server 30 is operated such that the number of view-prohibited items is increased or decreased, depending on the contract between the management provider and a service provider.

That is, upon a request for a view of the management information DB 300 from a user terminal 10 or a service provider server 20, the information management unit 304 of the management provider server 30 refers to the view control information table (for individual users) 3001 or the view control information table (for individual service providers) 3002, and transmits a response to the user terminal 10 or the service provider server 20, excluding the items, in the data of the service content, which correspond to view-prohibited items having been set in advance in the view control information tables 3001 or 3002.

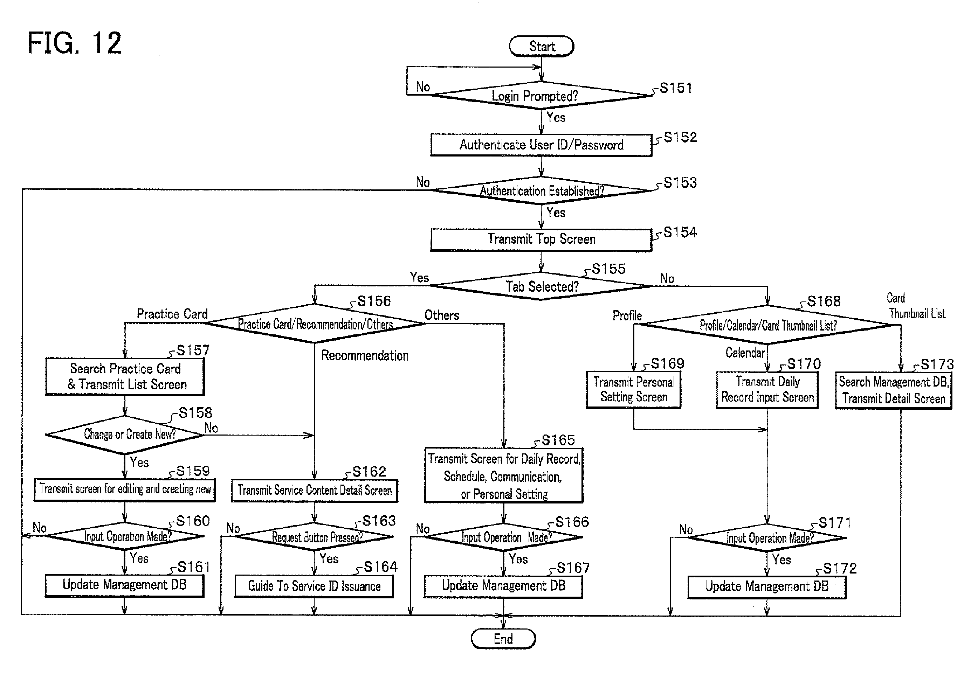

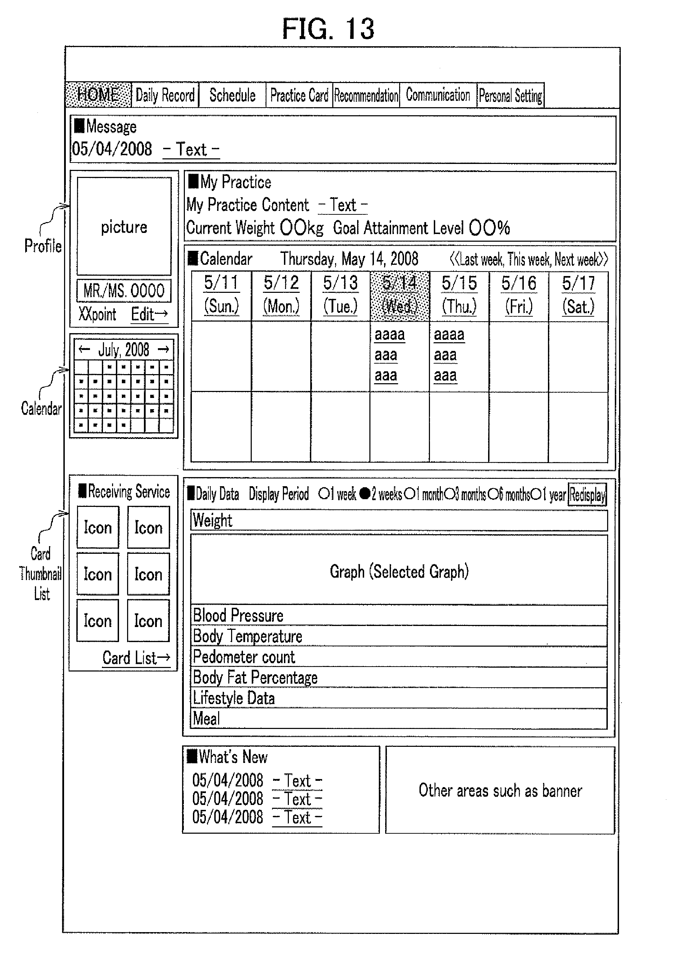

FIG. 12 is a flowchart showing the operation of the management information visualization device (management provider server 30) in the present embodiment, in particular to the user GUI providing unit 303. FIGS. 13, 14, and 15 are diagrams showing an example of the respective screen arrangements related to 'top screen', 'practice card list', and 'personal settings' , which are displayed on a user terminal 10.
The operation of the management information visualization device (management provider server 30) in the present embodiment shown in FIGS. 5 and 6 will be described in detail, referring to FIGS. 12 to 15.

First, through operation of a user terminal 10, a user accesses the site of the data collection and keeping service provider 3 for login (a request for viewing service information) (step S151 'Yes').
Upon receiving this, the management provider server 30 transmits information on a login screen, not shown, via the network 50 to the user terminal 10 having made the request, the login screen information being created by the user GUI providing unit 303. The user terminal 10 receives the login screen information; the display unit 102 displays the login screen information on a screen; and the data input unit 101 takes in the user ID and the password, which have been registered at the time of a service request and are input by the user, and transmits the user ID and the password via the network 50 to the management provider server 30.

Subsequently, on the side of the management provider server 30, the user management unit 301 performs authentication of the user ID and the password (step S152). If the authentication is established (step S153 'Yes'), then the user GUI providing unit 303 creates display information related to the top screen, shown in FIG. 13, and transmits, via the network 50, the display information to the user terminal 10 having made the request (step S154).
In creating the top screen, the user GUI providing unit 303 of the management provider server 30 searches in the management information DB 300 and obtains, by the information management unit 304, a list of services the user is currently receiving, the list including service IDs associated with the user ID.

On the top screen, the services the user is currently receiving from health service providers are represented by cards (icons, objects), and unique service IDs are given (assigned) to these cards.
As shown in FIG. 13, the upper portion of the top screen is allocated for the tabs of 'HOME', 'Daily Record', 'Schedule', 'Practice Card', 'Recommendation Card', 'Communication', 'Personal Settings', and when the user makes selection from these, the screen changes to a screen corresponding to a selected tab. Further, the left portion of the top screen is allocated for 'Profile', 'Calendar', and 'Card Thumbnail list', which are displayed n respective allocated areas.

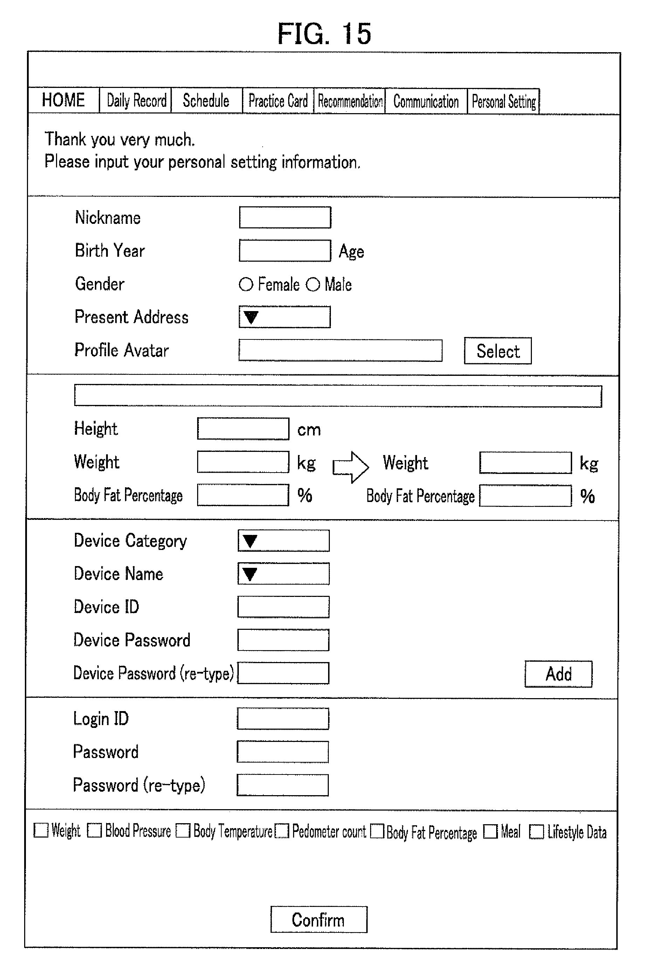

'Profile' shows the avatar of the user, and by operation of a button icon arranged and displayed below the avatar, the screen changes to 'Personal Setting Screen' shown in FIG. 15. 'Personal Setting Screen' will be described later. Into the column of name just below the avatar, a nickname is input instead of the real name of the user for protection of personal information.

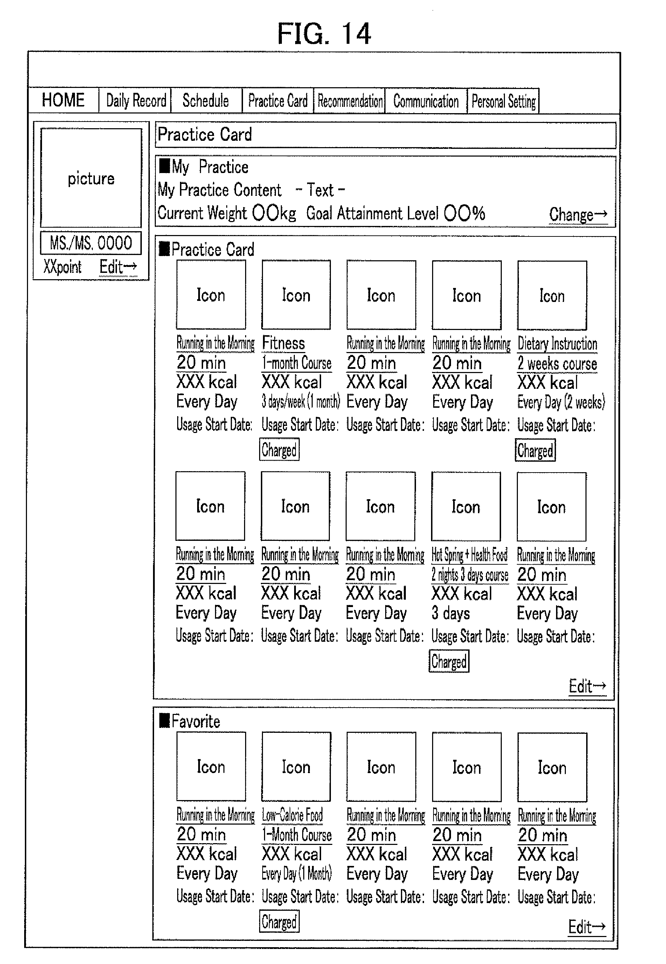

By pressing a date displayed on 'Calendar', the screen changes to a daily record input screen, not shown.
Incidentally, 'Card Thumbnail List' lists the contents of the services, which are associated with service IDs, currently received by the user, and displayed by icons. If any one of these icons is selected by operation, then the screen on the user terminal 10 changes to a detailed screen of a corresponding service. Further, if the button icon, which is arranged and displayed below 'Card Thumbnail List', is pressed, then the display screen on the user terminal 10 changes to 'Practice Card List Screen' shown in FIG. 14.

Still further, the middle of the top screen is allocated for areas of 'Message Column', 'My Practice Content', 'Calendar', 'Daily Data', 'New Information', and the like, which display the respective contents.
'Message Column' displays latest comment transmitted from a health service provider 2, and if there is no comment, even the frame itself is not displayed. Further, 'My Practice Content' displays a practice goal of the user, such as a goal of diet, by texts. Still further, if a date of 'Calendar' is selected by operation, then the display screen on the user terminal 10 changes to 'Daily Record Input Screen', not shown, and the content of a practice card and a schedule are displayed by texts in the blank. 'Daily Data Column' displays, for example, a graph of measured weight and the like in a designated period (one week to one year). Incidentally, 'New Information Column' displays an instruction comment or the like from health service providers, and the area arranged adjacent to 'New Information Column (What's New)' displays an advertisement banner or the like.

Returning to FIG. 12 to continue description, on the user terminal 10, after displaying the top screen transmitted from the management provider server 30, if the tab is selected by user operation (step S155 'Yes'), the input unit 101 of the user terminal 10 transmits information representing the kind of the tab to the management provider server 30 via the network 50, and the user GUI providing unit 303 of the management provider server 30 creates screen information according to the designated tab and transmits the screen information to the user terminal 10 having made the request.

For example, if the tab 'Practice Card' is selected (step S156 'Practice Card'), then the user GUI providing unit 303 searches in the management information DB 300 and creates, by the information management unit 304, screen information for a practice card list which is a list of services that have service IDs associated with the user ID and are currently received by the user, and transmits the screen information for the practice card list via the network 50 to the user terminal 10 having made the request (step S157).

FIG. 14 shows an example of the above-described 'Practice Card List Screen'. As shown in FIG. 14, 'Practice Card List Screen' is displayed being separated into a practice card area and a favorite area. In the practice card area, practice cards with assigned service IDs are displayed, and in the favorite area, practice cards for which service IDs have not yet been assigned are displayed, respectively by icons.

Further, practice cards include pay services, which are customized services, such as health instruction, received from a health service provider (for example, 'a course for obtaining a well-muscled body by fitness in a month (1 month course)', '2 weeks course of diet instruction', '2 nights 3 days course of hot spring + health food', '1 month course of low calorie food',), and free services, such as running in the morning, diet, and the like, for which a user himself/herself makes daily efforts and records data. Herein, it is assumed that practice cards are displayed with visual distinction by coloring of the icon frames or in another way. Returning to FIG. 12 to continue description, if any one of the displayed icons (refer to practice cards in FIG. 14) is pressed, then the display screen on the user terminal 10 changes to a corresponding detailed card screen, and if the displayed icon button 'Change' arranged in 'My Practice Column' in the top portion of the screen is selected by operation (step S158 'Yes'), then 'Practice Card Editing Screen', not shown, is transmitted from the management provider server 30 (step S159). Then, the user terminal 10 performs editing, following an input operation by the user (step S160, 'Yes'), transmits the edited contents via the network 50 to the management provider server 30, then the user GUI providing unit 303 updates the management information DB 300 under control by the information management unit 304 to reflect the edited contents (step S161). Incidentally, the case of creating a new card is similar with an exception of transmitting 'Practice Card Creating Screen' instead of the editing screen.

On the other hand, it will be assumed below that the user has selected by operation one of the icons displayed in the favorite area (step S158 'No'), and information on the detailed screen of service content of the corresponding service has been transmitted from the management provider server 30 to the user terminal 10 and displayed (step S162).
In this situation, if the user selects, by operation, 'Request Button' arranged in an area on the detailed screen of service content (step S163 'Yes'), the display screen on the user terminal 10 changes to 'service ID issuance screen' (not shown) made by the health service provider 2 who provides the corresponding service (step S164: guide to service ID issuance). This is a result of that, for a service which is in 'Practice Card List' displayed on the user terminal 10 and has been registered without being associated with a service ID, the user GUI providing unit 303 of the management provider server 30 transmits, to the user terminal 10, link information that guides to a service ID issuance screen to be transmitted by the service provider server 20 that provides a service, putting the link information into the information on a detailed screen of service content.

Incidentally, if the guide to the issuance of a service ID is performed, as described above (step S14), and the user requests the service (issuance of a service ID), then the icon displayed in the favorite area on the user terminal 10 is displayed, being moved from the favorite area to the practice card area.
It has been described about an example where the above-described guide to issuance of a service ID causes movement of an icon displayed in the favorite area, however, in another way, for example, by pressing a tab 'Recommendation' , an icon may be moved in a similar procedure(step S156 'Recommendation').

Incidentally, in the process of determining tab content in step S156, if the user selects another tab, such as 'Daily Record', 'Schedule', 'Communication', or 'Personal Setting' (step S156 'Others'), then the management provider server 30 (user GUI providing unit 303) creates information on an edit screen, individually matching the selected tab, and transmits the information to the user terminal 10 having made the request (step S165).
Then, following an operation of editing input by the user (step S166 'Yes'), the edit content is transmitted via the network 50 to the management provider server 30. Upon receiving the edit content, the management provider server 30 updates the management information DB 300 under control by the information management unit 304 to reflect the edit content (step S167).

On the other hand, in the process of determining tab selection in step S155, instead of tab selection, if any one of Profile, Calendar, and Card Thumbnail List is selected by operation ('No'), in a case, for example, that Profile is selected (step S168 'Profile'), then the user GUI providing unit 303 of the management provider server 30 transmits display information related to the personal setting screen shown in FIG. 15 (step S169). In a case that Calendar is selected by operation (step S168 'Calendar'), the user GUI providing unit 303 transmits information related to a daily record input screen, not shown (step S170), and in a case that Card Thumbnail List is selected by operation (step S168 'Card Thumb Nail'), the user GUI providing unit 303 searches in the management information DB 300 and transmits information related to a corresponding detail screen (step S173). Consequently, the user terminal 10 displays one of 'personal setting screen', 'daily record input screen', and 'detail screen'.

Herein, if an input operation has been made by the user, referring to respective screens (step S171 'Yes'), the user GUI providing unit 303 of the management provider server 30 takes in the input information via the 101 of the user terminal 10 and the network 50, and updates the management information DB 300 under control by the information management unit 304 (step S172).

Incidentally, the personal setting screen, shown in FIG. 15, is a screen for setting information related to the profile of the user (nickname, birth year, sex, current address, and profile avatar (selection of avatar)', data related to height, weight, body fat percentage, and the like as 'My Practice', device information (information on a testing device 40 including a device ID), and a user ID (password). The user operates the user terminal 10 to set the above-described various information, and can thereby enjoy the service, by the management provider server 30, of keeping and holding sensitive information, and the provision of visualized information, based on analysis of service information customized by a service provider server 20.
Incidentally, a service ID issued by a health service provider 2 may be set and registered, however, it is assumed herein that a device ID is commonly applied to a service ID.

As has been described above, a management information visualization device in the present embodiment is a management information visualization device (a management provider server 30) that is, for example as shown in FIG. 3, connected via a network 50 with user devices (user terminals 10) and service providing devices (service provider servers 20) that receive service provision requests from the user devices and obtains personal information including user identification information. The management information visualization device registers service information provided by the service providing devices, into a database (a management information DB 300) together with service content identification information (service IDs) assigned uniquely to each individual service that a user has contracted. The management information visualization device includes, for example with reference to FIGS. 4 and 5, an information registration management unit (for example, an information management unit 304) that registers service information transmitted from an above-described service providing device into the database, associating service information with user identification information (a user ID) and service content identification information which are related to this service information. The management information visualization device (the management provider server 30) also includes an information input/output unit (for example, a user GUI providing unit 303) that receives, from a user device, a request for viewing service information, the request being based on either user identification information or service content identification information or based on a combination of these; creates management information including a service content list associated with service content identification information and transmits the management information to the user device having made the request for viewing service information; and, when one from the service content list displayed on the user device being selected by operation via the user device, searches in the database and transmits corresponding service information to the user device having made the request for viewing service information.

### Modified Example

Herein, when a specific selection item (for example, a tab displayed on the top screen shown in FIG. 13) that is included in management information displayed on the user device is selected by operation, the information input/output unit may create a service content list (for example, the practice card list screen in FIG. 14) including service information that has been registered without being associated with service content identification information (service IDs), and transmit the service content list, for displaying, to the user device having made the request for viewing service information. In this modified example, it is not always necessary to display a card thumbnail list, as shown in FIG. 13, on the top screen (an entrance screen for an individual user). Incidentally, in this modified example, the screen in FIG. 14 corresponds to a list screen for an individual user, and in the foregoing embodiment, the screen in FIG. 13 corresponds to a list screen for an individual user.
Further, for service information in a service content list displayed on the user device, the service information having been registered without being associated with service content identifying information, the information input/output unit may set link information into an icon, the link information guiding to a service content identification information issuance screen to be transmitted by the service providing device which provides corresponding service information, and transmit the icon having the link information to the user device that has made the request for viewing service information.
For example, by setting link information into an icon (putting link information into an icon), as below, by the use of an anchor tag, guidance to a target screen is attained.
<a href=http://www.xxxxxx-sports-jim.com/index.html>
<img src=http://www.xxxxxx-xxx.com/xxxxx/xx-icon.gif>
</a>

By the above-described management information visualization device (the management provider server 30) in the present embodiment, a user can manage sensitive information of himself/herself visually by a service content list corresponding to service content information. Further, the user can recognize the contents of health management, which the user is currently practicing, by just viewing a service content list displayed on the user terminal 10. Still further, by just selecting, with operation, one from the service content list, the user can view the details of a content. Thus, user friendliness can be improved, and convenience can be obtained.
Further, the management information visualization device transmits the service content list, having service contents not yet associated with service content identification information be included in the service content list, and transmits then link information that guides to the service content identification information issuance screen to be transmitted by the service providing device that provides the service information, putting the link information into the service content list. In such a manner, the user can have a display of the service content identification issuance screen, as necessary, and obtain service content identification information. Thus, the management information visualization device can provide a more user-friendly user interface.
It is possible to easily guide to a target screen (site) by using the above-described anchor tag.

Still further, the management information visualization device (the management provider server 30) registers service information into the database without personal information of a user. Accordingly, it is not possible to recognize whose service information it is, which makes the strength of information security high and is also useful in the point of view of protection of personal information. Yet further, as coupling personal information with service content identification information is impossible without user identification information, the management information visualization device can block exchange of information between health service providers, enabling maintenance of security.

### Other Modified Examples

According to the management information visualization device (the management provider server 30) in the foregoing present embodiment, a user manages services that the user receives from a health service provider/providers by visualizing the services with cards on a WEB (World Wide Web) screen, however, these cards can also be distributed as a physical card recording medium in which service IDs are described. In this case, a password may be described in the card recording medium, obtained via the Web page of the health service provider 2, or obtained by a mail, etc. Further, the physical card recording medium also can be sold and bought. In this case, in a situation that, for example, a physical card recording medium with a service ID for receiving a service, for example, 'a course for obtaining a muscular body by fitness in a month' is available at a store, if a user requests the service (purchases the physical card recording medium), operates a PC (a user terminal 10) in a Web connection environment to access at home the server (a management provider server 30) of a data collection and keeping service provider 3, and registers the service ID described in the physical card recording medium, then the user thereby becomes able to receive the service by a health service provider 2 thereafter. Incidentally, physical card recording media may be distributed in a street as a trial version for promotion with limited validity.

Further, the functions of the user GUI providing unit 303 as an information input/output unit and the information management unit 304 as an information registration management unit in the management provider server 30 may be implemented all by software or at least a part of the functions may be implemented by hardware.
Data processing described below may be realized by one or a plurality of programs on a computer, or at least a part of the processing may be realized by hardware. Herein, for example, the information management unit 304 registers service information transmitted from a service providing device into the database (the management information DB 300), associating this service information with user identification information (a user ID) and service content identification information (a service ID) related to this service information. Further, the user GUI providing unit 303 receives, from a user terminal 10, a request for viewing service information, the request being based on either a user ID or a service ID or based on a combination of these; creates management information (for example, the top screen in FIG. 13) including a service content list associated with service IDs and transmits the management information to the user terminal 10 having made the request for viewing service information; and, when one from the service content list displayed on the user terminal 10 being selected by operation via the user terminal 10, searches in the management database 300 and transmits corresponding service information to the user terminal 10 having made the request for viewing service information.

Further, the information management system in the present embodiment is a system in which, for example as shown in FIG. 3, connected are, via a network 50, user devices (user terminals 10); service providing devices (service provider servers 20) that receive requests for service provision from the user terminals and obtains personal information including user identification information; and a management information visualization device (the management provider server 30) that registers service information provided by the service providing devices, together with service content identification information assigned uniquely to each individual service that a user receives, into a database (the management information DB 300). The service providing devices include a transmitting unit (for example, the data output unit 204 in FIG. 5) for transmitting the service information, when the service information being registered into the database of the management information visualization device, via the network to the management information visualization device, adding the service information with user identification information and service content identification information which are related to the service information. The management information visualization device includes an information registration management unit (for example, the information management unit 304 in FIGS. 4 and 5) that registers service information transmitted from a service providing device, associating the service information with user identification information and service content identification information which are related to this service information. The management information visualization device also includes an information input/output unit (for example, the user GUI providing unit 303) that receives, from a user device via the network, a request for viewing service information, the request being based on either user identification information or service content identification information or based on a combination of these; creates management information including a service content list including service information associated with service content identification information and transmits the management information to the user device having made the request for viewing service information; and, when one from the service content list displayed on the user device being selected by operation via the user device, searches in the database and transmits corresponding service information via the network to the user device having made the request for viewing service information.

In the information management system in the foregoing present embodiment, when service providing devices (service provider servers 20) provide various information services, such as health management of a user, testing, consulting, to user devices (user terminals 10), service contents are registered, being added with individual service content identification information, and further the management information visualization device (the management provider server 30) registers and manages the service content identification information, associating the service content identification information with user identification information. Consequently, by designating either user identification information or service content identification information, or by designating a combination of these, as a search condition through operation of a user terminal 10, a user can view management information (sensitive information) on the user collected by the management provider device and service information, such as health instruction, registered by a service provider server 20.

Further, in the information management system in the foregoing present embodiment, sensitive information of a user himself/herself is visually displayed on a user terminal 10, as shown in FIGS. 13 and 14 for example, via cards corresponding to individual service content identification information, which enables the user to manage service information, using a user-friendly interface. Still further, herein, the management provider server 30 keeps management information, such as sensitive information, without personal information of users, which attains a high security strength with respect to personal information and enables the data collection and keeping service provider 3 to avoid risks in a point of view of protection of personal information as well.

The method for visualization of management information in the present embodiments is a method for an information management system in which connected are via a network 50, as shown in FIG. 3 for example, testing devices 40 for measuring sensitive information on users, user devices (user terminals 10), service providing devices (service provider servers 20) that receive service requests from user terminals 10 and obtain personal information including user identification information, and a management information visualization device (a management provider server 30) that registers service information provided by the service provider servers 20 together with service content identification information uniquely assigned to individual services received by the users into a management information DB 300.

For example, in the sequence diagram in FIG. 4, the method for visualization of management information includes the steps of:
(1) A step (S112) of issuing, by the management provider server (the management information visualization device) 30, one or more pieces of service content identification information, based on a service request from a service providing server (service providing device) 20;
(2) A step (S114) of assigning, by the service provider server 20, to a service request from a user terminal (user device) 10, one of the service IDs (the pieces of service content identification information) issued by the management provider server 30;
(3) A step (S122), by the management provider server 30, of collecting sensitive information on each individual user measured by a testing device 40 and accumulating the sensitive information on the each individual user into the management information DB (database) 300, based on device identification information uniquely assigned to the each individual testing device 40;
(4) A step (S130 to S135) of generating, by a service provider server 20, service information customized for each individual user from the sensitive information of the user, based on the service ID and with reference to the management information DB 300, and storing the service information into the management information DB 300 of the management provider server 30, associating the service information with the service ID assigned to the user;
(5) A step (S127, S128), by the management provider server 30, of receiving a request for viewing service information from a user terminal 10, based on either a user ID (user identification information) or a service ID, or based on a combination of these, creating management information including a service content list associated with service IDs and transmitting the management information to the user terminal 10 having made the request for viewing service information, and, when one from the service content list displayed on the user terminal 10 being selected by operation via the user terminal 10, searching in the management information DB 300 and transmitting corresponding service information via the network to the user terminal 10 having made the request for viewing service information; and
(6) A step (S129), by the user terminal 10, of receiving the service information transmitted from the management provider server 30 and displaying the service information on a screen.

According to the method for visualization of management information in the present embodiment for an information management system, when a health service provider 2 provides various services, such as health management of an end user 1, testing, consulting, the service contents are registered, being added with individual service IDs, and further a data collection and keeping service provider registers the service IDs, associating the service IDs with user IDs. Consequently, by designating either a user ID or a service ID, or by designating a combination of these, as a search condition, the end user 1 can view service information registered by the health service provider 2. Further, the end user 1 can visually manage sensitive information of himself/herself, as shown in FIGS. 13 and 14 for example, via cards corresponding to individual service IDs. Consequently, operability is improved by a user-friendly user interface. Further, as the data collection and keeping service provider 3 keeps sensitive information and the like without personal information of users, the data collection and keeping service provider 3 is unable to know to whom each sensitivity information belongs. Consequently, it is possible to attain a high strength of information security and enable the data collection and keeping service provider 3 to avoid risks in a point of view of protection of personal information as well.

An embodiment according to the present invention has been described above, however, the invention is not limited thereto and can be carried out in a range without departing from the spirit of the invention. The concrete configuration of hardware and software can be changed or modified, as appropriate, in a range without departing from the spirit of the invention. For example, a service provider server 20 may also have the functions of a management provider server 30, and the reverse may be carried out.

## Claims

1. A method for supporting information management of a user, using user/service-content association information in which association relationship is registered between user identification information for uniquely identifying a user and service content identification information for uniquely identifying, for each service, from which service provider and to which user a service received by the user is provided, the method comprising:
at a management information visualization device,
obtaining via a network the user identification information having been input from a user device operated by the user;
extracting service content identification information corresponding to the obtained user identification information, referring to the user/service-content association information stored in a storage device;
creating a list screen information for personal use in which the extracted individual service content identification information is assigned to one icon or one card, transmitting the list screen information via the network to the user device, and having the user device display the list screen information on a screen such that the each icon or the each card is selectable;
detecting via the network a selection by the user of an icon or a card disposed on the list screen for personal use displayed by the user device; and
upon the detection, making via the user device certain service information viewable, the certain service information corresponding to the service content identification information assigned to the selected icon or the selected card.

2. The method for supporting information management according to claim 1, further comprising:
at the information visualization device when the user identification information is obtained via the network,
creating screen information for displaying an entrance screen for personal use by the user who is uniquely identified by the obtained user identification information, transmitting the screen information via the network to the user device, and having the user device display the screen information; and
upon detection via the network that an operation for having the list screen for personal use displayed has been made on the displayed entrance screen for personal use, transmitting the list screen information for personal use to the user device and having the user device display the list screen information.

3. A management information visualization device that is connected via a network with a user device and a service providing device which receives a request for providing a service from the user device and obtains personal information including user identification information, wherein the management information visualization device registers service information provided by the service providing device into a database together with service content identification information that is individually assigned to each service used by a user and unique to the user and the service, the management information visualization device comprising:
an information registration management unit that registers service information transmitted from the service providing device into the database, associating the service information with user identification information and service content identification information which are related to the service information; and
an information input/output unit that receives, from the user device, a request for viewing service information, the request being based on either user identification information or service content identification information or based on a combination of the user identification information and the service content identification information; creates management information including a service content list associated with service content identification information and transmits the management information to the user device having made the request for viewing service information; and, when one from the service content list displayed on the user device being selected by operation via the user device, searches in the database and transmits corresponding service information to the user device having made the request for viewing service information.

4. The management information visualization device according to claim 3,
wherein, when a specific selection item that is included in the management information displayed on the user device is selected by operation, the information input/output unit creates a service content list information including service information that has been registered without being associated with service content identification information, and transmits the service content list information, for displaying, to the user device having made the request for viewing service information.

5. The management information visualization device according to claim 4,
wherein, with respect to service information that has been registered without being associated with service content identifying information among service information in the service content list information displayed on the user device, the information input/output unit puts link information into the service content list information, the link information guiding to a service content identification information issuance screen to be transmitted by the service providing device which provides service information, and transmits the service content list information to the user device that has made the request for viewing service information.

6. An information management system, comprising:
a user device;
a service providing device that receives a request for service provision from a user device and obtains personal information including user identification information; and
a management information visualization device that registers service information provided by the service providing device, into a database together with service content identification information individually and uniquely assigned to each service that a user receives,
wherein the user devices, the service providing device, and the management information visualization device are connected via a network,
wherein the service providing device includes:
a transmitting unit for, when the service information being registered into the database of the management information visualization device, transmitting the service information via the network to the management information visualization device, adding the service information with user identification information and service content identification information which are related to the service information,
and wherein the management information visualization device includes:
an information registration management unit that registers service information transmitted from the service providing device, associating the service information with user identification information and service content identification information which are related to the service information; and
an information input/output unit that receives, from a user device via the network, a request for viewing service information, the request being based on either user identification information or service content identification information or based on a combination of the user identification information and the service content identification information; creates management information including a service content list including service information associated with service content identification information and transmits the management information to the user device having made the request for viewing service information; and, when one from the service content list displayed on the user device being selected by operation via the user device, searches in the database and transmits corresponding service information via the network to the user device having made the request for viewing service information.

7. The information management system according to claim 6, wherein the user device:
receives the management information including the service content list transmitted from the management information visualization device via the network;
displays the management information on a screen to prompt a user for selection with operation;
obtains information on service content selected with operation by the user;
transmits the information via the network to the management information visualization device; and
receives corresponding service information transmitted via the network from the management information visualization device and displays the service information on a screen.

8. A method for visualization of management information in an information management system having a testing device for measuring sensitive information on a user, a user device, a service providing device that receives a service request from the user device and obtains personal information including user identification information, and a management information visualization device that registers service information provided by the service providing device, into a database together with service content identification information individually and uniquely assigned to each service that a user receives, wherein the testing device, the user device, the service providing device, and the management information visualization device are connected via a network, the method comprising the steps of:
issuing, by the management information visualization device, one or more pieces of service content identification information, based on a service request from the service providing server;
assigning, by the service providing device, to a service request from a user device, one piece of service content identification information issued by the management information visualization device;
collecting, by the management information visualization device, of sensitive information on each individual user measured by a testing device and accumulating the sensitive information on the each individual user into the database, based on device identification information uniquely assigned to the each individual testing device;
generating, by the service providing device, service information customized for each individual user from the sensitive information of the user, based on the service content identification information and with reference to the database, and storing the service information into the database of the management information visualization device, associating the service information with the service content identification information assigned to the user;
receiving, by the management information visualization device, of a request for viewing service information from a user device, based on either user identification information or service content identification information, or based on a combination of the user identification information and the service content identification information, creating management information including a service content list associated with service content identification information and transmitting the management information to the user device having made the request for viewing service information, and, when one of the service content list displayed on the user device being selected by operation via the user device, searching in the database and transmitting corresponding service information via the network to the user device having made the request for viewing service information; and
receiving, by the user device, the service information transmitted from the management information visualization device and displaying the service information on a screen.
